# EUROPEAN PATENT APPLICATION

(11) **EP 4 803 038 A1**
(43) Date of publication of application: **09.09.2026**
(21) Application number: 25305282.3
(22) Date of filing: 03.03.2025
(51) Int. Cl.: A61F 2/16, B29D 11/00, B82Y 30/00, B82Y 40/00, G02B 1/00

(54) **POLARIZATION-INSENSITIVE INTRAOCULAR LENS**

(71) Applicant: Centre national de la recherche scientifique, 75016 Paris (FR); Université Paris Cité, 75006 Paris (FR); Fondation Adolphe de Rothschild, 75019 Paris (FR); Università degli Studi di Brescia, 25121 Brescia (IT)
(72) Inventor: LEO, Giuseppe, 94120 Fontenay sous Bois (FR); DE ANGELIS, Costantino, 25100 Brescia (IT); GATINEL, Damien, 75006 Paris (FR)
(74) Representative: IPAZ

(57) **Abstract**

The present invention relates to a polarization-insensitive intraocular lens (100) comprising an anterior part (110), a posterior part (120), an equatorial plane (130), and two optical loops (190), wherein the anterior part (110) and the posterior part (120) are connected through the equatorial plane (130) and the optical loops (190) are connected with the equatorial plane (130), characterized in that said intraocular lens comprises moreover a solid-state dielectric metasurface structure (140), arranged on the equatorial plane (130), said metasurface structure comprising a plurality of vertical and preferably cylindrical nano-pillars (150) of different sections (S).

The present invention also concerns a method for manufacturing such an intraocular lens (IOL).

## Description

### FIELD OF THE INVENTION

The present invention relates to a polarization-insensitive intraocular lens and a method for manufacturing the same. The intraocular lens according to the invention is intended to be implanted into the interior of the human eye and includes a pseudophakic intraocular lens for replacing the natural lens of a cataract patient and a phakic intraocular lens to realize a refractive correction function.

### BACKGROUND

Over the past few decades, intraocular lenses (IOLs) have revolutionized vision correction, providing millions of people worldwide with convenient, effective, and customizable solutions. Continuous advancements in materials and optical designs have allowed researchers and manufacturers to address various visual impairments and challenges.

However, current IOLs still face limitations in terms of multifocality, extended depth of focus, and higher-order aberration correction. The next frontier in IOL development lies in the customization of lenses based on individual ocular characteristics, ensuring optimal visual outcomes for each patient.

Several clinical concerns have gained prominence in ophthalmology, driven by lifestyle changes that contrast with the evolutionary adaptation of the human eye. These issues include the unprecedented global rise in myopia, post-cataract surgery discomfort, and keratoconus-related vision challenges. Traditional solutions such as spectacles, contact lenses, and standard IOLs are not always sufficient to address these conditions, highlighting the need for advancements in ophthalmologic diagnostics, surgical techniques, and therapeutic interventions.

An IOL is an artificial lens implanted in the eye either alongside the natural lens (to correct refractive errors such as myopia and hyperopia) or as a replacement for the natural lens in cataract surgery. It is referred to as phakic IOL and pseudophakic IOL, respectively.

Monofocal IOLs provide clear vision at a single focal point, either near or far.

Multifocal IOLs are designed to compensate for the loss of natural accommodation, allowing vision at multiple distances. These lenses achieve multifocality through varying surface curvatures or by integrating a Fresnel lens structure into a monofocal design.

While multifocal IOLs improve vision across different distances, they can sometimes cause visual disturbances, such as glare and halos, due to light scattering at discontinuities within the lens structure.

Research suggests that metalenses (MLs) can provide a breakthrough by reducing IOL thickness, enhancing multifocal or extended-depth-of-focus performance, and enabling personalized wavefront shaping for improved vision correction.

Document WO2023274186 A1 discloses an IOL based on a metasurface, including a front optical lens, a rear optical lens, an equatorial plane, and two optical loops; where the front optical lens and the rear optical lens are connected through the equatorial plane, the optical loops are connected with the equatorial plane. A metasurface structure is arranged on the equatorial plane, said metasurface structure including a plurality of nanostructure units made of PMMA (Polymethyl Methacrylate), with a phase distribution of a planar axicon lens.

However, the intraocular lenses according to Document WO2023274186 (A1) form an axicon that does not correct aberrations.

The paper "Geometric-phase intraocular lenses with multifocality" Seungmin Lee et al. Light Science & Applications (2022) 11:320, demonstrates a new type of multifocal and extended depth of focus (EDOF) intraocular lenses (IOLs) embedding µm-thin geometric phase (GP) lens layers. As an emerging approach for lens phase design, the GP modulated IOLs outperform conventional diffractive IOLs in multifocality while completely avoiding the clinically undesirable demand for additional surface patterns to standard monofocal IOL designs.

However, the intraocular lenses according to this paper are sensitive to polarization because they are made of oriented liquid crystals.

An objective of the present disclosure is to provide an IOL that presents better control of halos, starbust and glare.

Another objective of the present disclosure is to provide an IOL based on a metasurface to implement new functionalities in terms of aberrations correction and arbitrary wavefront shaping.

Another objective of the present disclosure is to provide phakic lens with smaller thickness than current IOLs.

### SUMMARY OF THE INVENTION

The object is achieved according to the invention by a polarization-insensitive intraocular lens comprising an anterior part, a posterior part, an equatorial plane, and two optical loops, wherein the anterior part and the posterior part are connected through the equatorial plane and the optical loops are connected with the equatorial plane. The intraocular lens comprises moreover a solid-state dielectric metasurface structure, arranged on the equatorial plane, said metasurface structure comprising a plurality of vertical and preferably cylindrical nano-pillars of different sections.

Optionally, each nano-pillar has a circular section with a diameter of between 50 and 500 nm and acts as a nano-pillar enabling the propagation of the fundamental mode. Any diameter choice maps into one value for the effective index *n*_{eff}, which is comprised between the refractive indices of the encapsulating polymer *n*₀ and the nano-pillar *n*_{g} (*n*₀ < *n*_{eff} < *n*_{g}). *n*_{eff} can be defined as the ratio between the speed of light in vacuum and the phase velocity of the guided mode.

Optionally, each nano-pillar has a height of between 100 and 1000 nm. The phase delay between output and input light being proportional to the product between *n*_{eff} and the nano-pillar height, the latter is chosen as the minimum value that grants for a 2*π* relative dephasing between the nano-pillars with smallest and largest diameters. This minimization is beneficial for nano-pillar fabrication.

Optionally, the interaxial distance between two adjacent nano-pillars is in a range of 200 and 1200 nm. Such distance is chosen so as to control the coupling between nano-pillars and maximise the metasurface efficiency.

Optionally, the dielectric metasurface structure comprises between 10⁶ and 10⁸ nano-pillars. Given the typical diameter of the whole IOL (≈ 6 mm), the choice of this number stems from a compromise between the factors relating to the interaxial distance.

Optionally, the plurality of nano-pillars is arranged in a range of 10³ and 10⁴ concentric rings. Given the typical diameter of the whole IOL (≈ 6 mm), the choice of this number stems from a compromise between the factors relating to the interaxial distance.

Optionally, the plurality of vertical and preferably cylindrical nano-pillars is made of Titanium Dioxide (TiO2) or of Gallium Nitride (GaN). The choice of the nano-pillar material is dictated by the requirement of transparency in the visible spectral range, its technological maturity, and the maximization of its refractive index so as to minimize the nanofabrication-sensitive aspect ratio between the height and the diameter of the nano-pillars.

Optionally, a refractive index of each nano-pillar is in a visible wavelength range of 2.1 and 2.7.

The invention relates also to a method for manufacturing a polarization-insensitive intraocular lens comprising an anterior part, a posterior part, an equatorial plane, and two optical loops, the anterior part and the posterior part being connected through the equatorial plane and the optical loops being connected with the equatorial plane, said intraocular lens comprising moreover a solid-state dielectric metasurface structure arranged on the equatorial plane, said metasurface structure comprising a plurality of vertical and preferably cylindrical nano-pillars of different sections (S), wherein the method comprises the following steps :
- Providing a first disc of solid-state biocompatible material;
- Forming on one side of said first disc the solid-state dielectric metasurface structure by depositing the plurality of vertical and preferably cylindrical nano-pillars of different sections (S);
- Encapsulating the solid-state dielectric metasurface structure in a second disc of solid-state biocompatible material, the first disc and the second disc being thus assembled together;
- Machining the first disc and the second disc according to a predefined shape to form the intraocular lens.

Optionally, the step of encapsulation comprises:
- Coating the solid-state dielectric metasurface structure with a liquid-state biomaterial;
- Covering the solid-state dielectric metasurface structure coated with the liquid-state biomaterial, with the second disc of solid-state biocompatible material.
- Curing the liquid-state biomaterial.

Optionally, the plurality of vertical and preferably cylindrical nano-pillars is made of Titanium Dioxide (TiO2) or of Gallium Nitride (GaN).

Optionally, the plurality of vertical and preferably cylindrical nano-pillars is formed by Electron Beam Lithography, Atomic Layer Deposition, Molecular Beam Epitaxy, or ICP-Reactive Ion Etching.

Optionally, each nano-pillar has a height of between 100 and 1000 nm, a circular section with a diameter of between 50 and 500 nm, the interaxial distance between two adjacent nano-pillar being in a range of 200 and 1200 nm.

Optionally, the solid-state dielectric metasurface structure comprises between 10⁶ and 10⁸ nano-pillars.

Optionally, the plurality of nano-pillars is arranged in a range of 10³ and 10⁴ concentric rings.

Optionally, a refractive index of each nano-pillar is in a visible wavelength range of 2.1 and 2.7.

### BRIEF DESCRIPTION OF THE FIGURES

Preferred embodiments of the invention are disclosed in the following description and the accompanying drawing which are merely illustrative of such invention.
Figure 1 shows a sagittal cross-section of a human eye.
Figure 2 shows a perspective view of an intraocular lens according to one embodiment of the invention.
Figure 3 illustrates the optical mechanisms of metasurfaces.
Figure 4 shows a perspective view of an intermediate state of an intraocular lens during a manufacturing process according to the invention.

### DETAILED DESCRIPTION

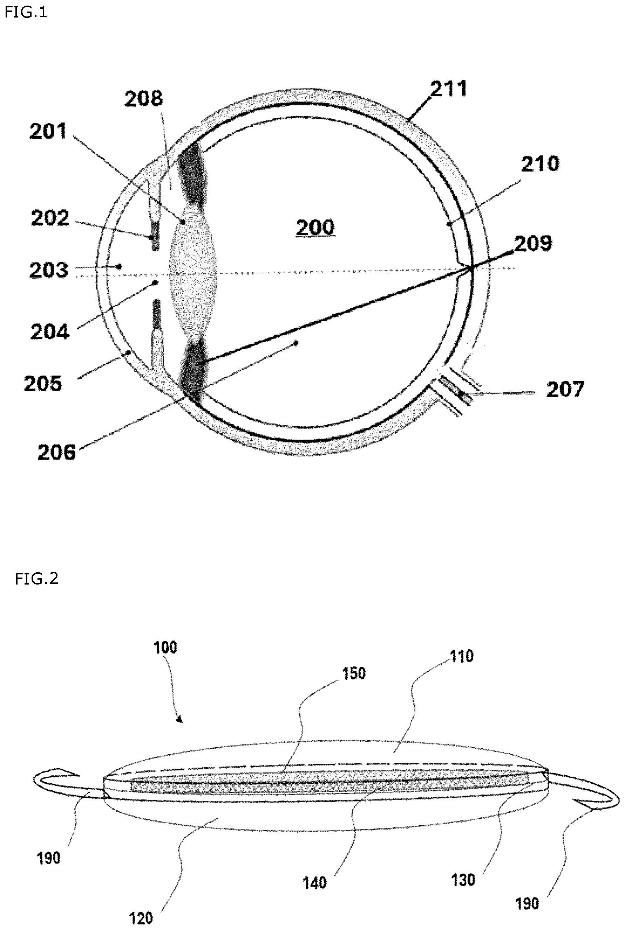

Figure 1 shows a sagittal cross-section of a human eye 200 constituting a complex organ that enables vision by detecting light and converting it into electrical signals sent to the brain. Below is a detailed breakdown of its anatomy and function:

It comprises external structures such as the sclera 211 as an outer protective layer of the eye, the cornea 205 as a transparent, dome-shaped front part of the eye that helps focus incoming light, the iris 202 as a colored part of the eye that regulates the amount of light entering the eye by adjusting the size of the pupil, the pupil 204 as a black circular opening in the center of the iris that allows light to pass through.

It comprises also internal structures, such as natural lens 201 as transparent, flexible structure behind the pupil that changes shape to focus light onto the retina, ciliary muscles 209 to control the shape of the natural lens for near or distant vision, vitreous humor 206 as a gel-like substance that fills the space between the lens and the retina, the retina 210 as a thin layer of light-sensitive cells that convert light into electrical signals. The optic nerve 207 transmits visual information from the retina to the brain.

The anterior chamber 203 and posterior chamber 208 are two fluid-filled spaces in the human eye that help maintain intraocular pressure and provide nutrients to eye structures.

The anterior chamber 203 is located between the cornea and the iris and is filled with aqueous humor. It provides oxygen to the cornea and lens and helps remove waste products from the eye.

The posterior chamber 208 is located between the iris and the natural lens and ciliary body. Also filled with aqueous humor, produced by the ciliary processes, it provides aqueous humor to the anterior chamber through the pupil and plays a role in maintaining intraocular pressure.

Referring to Figure 2 that shows a perspective view of an intraocular lens 100 according to one embodiment of the invention, said intraocular lens 100 comprises an anterior part 110, a posterior part 120, an equatorial plane 130, and two optical loops 190 (haptics).

The anterior part 110 refers to the part positioned towards the front of the eye, while the posterior part 120 refers to the part positioned on the side of the optic nerve, when the intraocular lens is implanted.

The anterior part 110 and the posterior part 120 are connected through the equatorial plane 130 and the optical loops 190 are connected with the equatorial plane 130.

The optical loops 190 refer to a specialized haptic design that helps position and stabilize the lens inside the eye after implantation. They help in preventing decentration, tilt, or rotation of the IOL.

According to the invention, the intraocular lens 100 comprises moreover a solid-state dielectric metasurface structure 140, arranged on the equatorial plane 130. The metasurface structure comprising a plurality of vertical and preferably cylindrical nano-pillars 150 of different sections S.

Refractive and diffractive optical components shape the wavefront by manipulating the phase shift accumulated during light propagation. Nanostructures engraved at the interface between two materials introduce an additional surface polarization, expanding the scope of creativity toward new non-refractive components. These optical interfaces, or metasurfaces, often exhibit unexpected properties and performances that can be advantageous for applications in embedded optics.

To control light beams, traditional components rely on the accumulated phase delay during propagation within the material. The "refractive" technology is based on expertise in shaping, polishing, and surface control. In a prism or a lens, the thickness t traversed in a material of refractive index n varies continuously to increase the optical path by the amount Δ=(n-1)t compared to propagation in air.

The optical function of a component is thus entirely determined by its intrinsic properties: shape and refractive index.

While refractive technology appears to have been fully exploited, the development of several innovative concepts in nanophotonics now introduces new degrees of freedom and opens up new possibilities.

Nanotechnologies enable the design of a new class of materials called "metamaterials" which allow for the conception of novel optical effects, both unusual and potentially revolutionary, such as negative refraction, and the development of superlenses, just to mention a few. Controlling light propagation in these 3D metamaterials requires structuring at a sub-wavelength scale in all three spatial dimensions, making the technological challenge particularly demanding.

Metasurfaces, which are 2D optical elements composed of arrays of nano-elements such as nanostructures, nanopillars, and other dielectric or metallic particles, circumvent the need for long propagation paths by introducing abrupt phase, amplitude, and/or polarization changes along the optical path at a thickness scale comparable to the wavelength. These structures offer great flexibility in wavefront control, eliminate the efficiency losses of conventional diffractive components, and revolutionize fabrication methods.

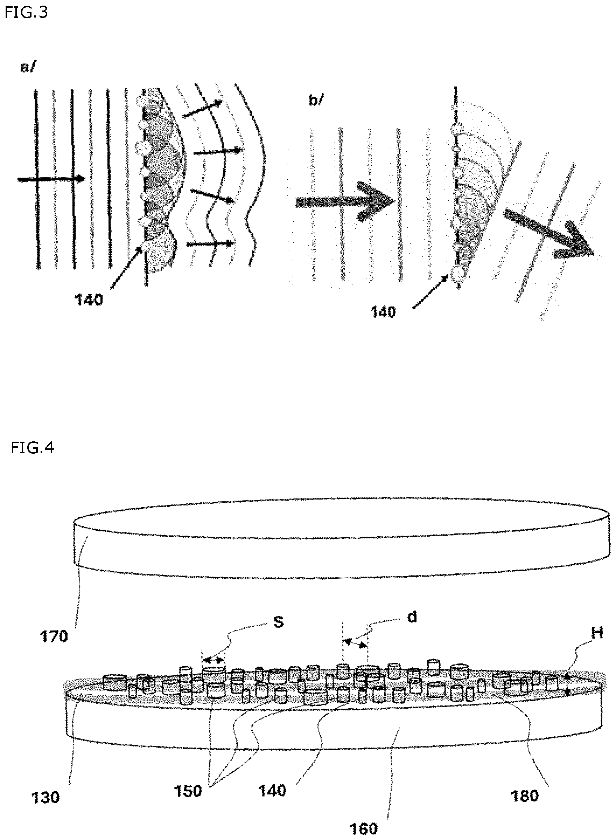

Figure 3 illustrates in panel a/ the operation of a metasurface based on Huygens' principle, which states that every point in space receiving an electromagnetic wave becomes a secondary source of new spherical waves. The electromagnetic field at any given point in space is obtained by summing the contributions of all secondary sources. This principle assumes that the field radiated by secondary sources depends solely on the wave state of the incident field.

Figure 3 illustrates in panel b/ that with a metasurface, local modifications of the radiated field occur at a sub-wavelength scale. Thus, when a beam scatters on an array of nanostructures designed with a phase shift varying linearly between neighboring nano-elements, the transmitted wavefront is modified according to the relation:
${n}_{t} sin {θ}_{t} - {n}_{i} ⋅ sin {θ}_{i} = \frac{{λ}_{0}}{2 π} ⋅ \frac{\partial φ}{\partial z}$
where *λ*₀ represents the wavelength in vacuum, *n_{i,t}* and *θ_{i,t}* are the refractive index and the incidence/transmission angle, respectively. In this example, the introduced phase gradient $\frac{\partial φ}{\partial z}$ along the intersection of the plane of incidence with the metasurface is constant.

Dielectric cavities can exhibit high-quality photonic resonances, such as Fabry-Pérot resonances or whispering gallery modes, initially discovered in acoustics by Lord Rayleigh. By reducing their size to a sub-wavelength scale and increasing the refractive index (e.g. SiO₂, n≈1.45n, Si, n≈3.4n), it becomes possible to excite different optical modes.

Unlike metallic particles, dielectric, semiconductor (Si, Ge, GaAs, GaN...), or oxide-based particles (TiO₂, TeO₂...) exhibit both electric and magnetic responses of similar amplitudes. A key application of this phenomenon was the observation of specific light scattering conditions, known as Kerker conditions, in non-magnetic materials (with magnetic permeability equal to that of vacuum).

For the purpose of the invention, titanium dioxide (TiO₂) or Gallium Nitride (GaN) are excellent candidate materials because of high refractive index (n = 2.1-2.7 for both materials throughout the visible range) and transparency at visible wavelengths.

Kerker, Wang, and Giles predicted in 1983 the absence of backscattering in the direction of the incident wave when the magnetic permeability equals the dielectric permittivity, and a maximum backscattering for another condition involving both permeability and permittivity.

Exciting both electric and magnetic Mie dipole modes allows these conditions to be met with a dielectric material lacking magnetism. When the electric fields emitted by the two dipoles constructively interfere in the forward direction but destructively interfere in the backward direction, backscattering is suppressed (first Kerker condition). By adjusting the frequency, it is possible to meet the second Kerker condition, where forward scattering is minimized.

These forward or backward scattering properties have been quickly exploited for designing structured surfaces with resonators, utilizing backscattering for reflective surfaces (mirrors) or forward scattering for transparent surfaces. Particular attention has been given to Huygens sources emitting in the forward direction like electric and magnetic dipoles.

Dielectric Mie resonators present new opportunities for creating Huygens metasurfaces, which scatter radiation forward and exhibit high transmission while modulating the wave phase through the excitation of both electric and magnetic resonances. Combining these two resonances enables a full 2π phase excursion required for metasurface design.

To arbitrarily manipulate an optical wavefront, a dielectric MS must locally control the phase shifts from 0 to 2*π*. To this end, three main strategies can be adopted, based on:
- the exploitation of a geometrical phase provided by nano-pillars of identical shape and size but different orientation;
- vertical nano-pillars of different sections (and therefore different effective index); and
- Huygens nano-disks, with two frequency-degenerated electric and magnetic dipole resonances. However, the geometric phase only works with circularly polarized light, while Huygens nano-disks have a very narrow spectral band and present fundamental limitations for beam shaping.

Thus, this invention relies on vertical nano-pillars 150, presenting sections S that may differ from each other.

Advantageously, nano-pillars 150 present a circular section, which provides them with a polarization-insensitive local response.

Propagation in each of the nano-pillars should be taken into account, as well as interactions between neighbouring elements.

Two conflicting considerations come into play in a quasi-local design: on the one hand, the wavefront must be sampled finely enough to faithfully follow the desired shape, leading to an increase in the number of nano-elements, and on the other hand, the coupling effects between neighbouring nano-elements must be minimized, leading to a reduction in the number of these elements.

A compromise should be obtained, for which each nano-pillar a well-defined mode. The desired phase is suitably sampled, and propagation takes place in each nano-pillar independently of what is happening in its neighbours. In this case, the mode guided in each nano-pillar is virtually independent of the angle of incidence, producing the same phase shift profile whatever the shape of the incident wavefront.

For applications relevant to human vision, and as depicted in Figure 4, each nano-pillar 150 of the intraocular lens according to the invention has preferably a height H of between 100 and 1000 nm.

Similarly, each nano-pillar 150 has preferably a circular section S with a diameter D of between 50 and 500 nm.

In the same way, the interaxial distance d between two adjacent nano-pillars is preferably in a range of 200 and 1200 nm.

In the same way, the dielectric metasurface structure comprises preferably between 10⁶ and 10⁸ nano-pillars.

In the same way, in the case of both monofocal or multifocal corrections, the plurality of nano-pillars is arranged in a range of 10³ and 10⁴ concentric rings.

The material constituting the anterior part 110 and the posterior part 120 must be biocompatible, ensuring minimal reaction with ocular tissues, and maintaining high optical precision.

Historically, poly(methyl methacrylate) (PMMA) was the standard for IOL materials due to its excellent biocompatibility. However, its inability to fold limited its practicality.

For the purpose of the invention, foldable acrylic materials have become the preferred choice, as they allow micro-incision surgery and reduce thus trauma and recovery time.

Turning now to the method for manufacturing a polarization-insensitive intraocular lens 100 according to the invention, the method comprises the steps described hereinafter.

According to a first step, a first disc 160 of solid-state biocompatible material is provided, said first disc being obtained for example by molding a foldable acrylic.

According to a second step, a solid-state dielectric metasurface structure 140 is formed on one side of the first disc by depositing a plurality of nanopillars (also called vertical nano guides) 150, of different sections S. It goes without saying that the metasurface shape needs to be defined in advance according to the patient's needs, insofar as nanopillars are high-aspect-ratio (tall and thin) nanostructures arranged in a 2D array. They function as nanoresonators that modulate the properties of incident light.

Nanopillars work based on:
- Mie Resonances
   Dielectric nanopillars support electric and magnetic dipole resonances (Mie modes) that tailor light interaction. Adjusting nanopillar geometry tunes these resonances, controlling phase and amplitude.
- Geometric Phase (Pancharatnam-Berry Phase)
   Asymmetric nanopillars can impart phase shifts dependent on their rotation angle, enabling beam deflection, holography, and vortex beam generation.
- Effective Refractive Index
   Varying nanopillar size and spacing creates a gradient index profile, allowing beam focusing (similar to gradient-index optics).

According to a third step, the solid-state dielectric metasurface structure 140 is encapsulated in a second disc 170 made of solid-state biocompatible material, the first disc 160 and the second disc 170 being thus assembled together.

According to a fourth step, the first disc 160 and the second disc 170 assembled together are machined according to a predefined shape to form the intraocular lens 100.

The step of machining can be a step of mechanical, laser or ultrasonic micromachining, which means high-precision material removal techniques used to create micro-scale structures with dimensions from the micrometer (µm) to the millimeter (mm) range.

The step of machining can be also a step of cryolathing, that means an ultra-precision machining technique that involves cooling the material to cryogenic temperatures (typically below -150°C) during the lathing process. This method improves the machinability of materials, especially soft, ductile, or thermally sensitive materials.

The intraocular lens 100 can moreover undergo polishing to enhance surface smoothness and reduce light scattering.

According to one preferred embodiment, the step of encapsulation comprises the following sub-steps.

According to a first sub-step, the solid-state dielectric metasurface structure 140 is coated with a liquid-state biomaterial 180.

According to a second sub-step, the coated solid-state dielectric metasurface structure 140 is covered with the second disc 170 of solid-state biocompatible material.

According to a third sub-step, the liquid-state biomaterial 180 is cured.

Turning now to the formation of the plurality of vertical and cylindrical nano-pillars 150, several techniques can be used.

TiO2 nanopillars can be deposited by Atomic Layer Deposition. Atomic Layer Deposition (ALD) is a thin-film deposition technique based on sequential, self-limiting chemical reactions. It enables precise control over film thickness at the atomic scale, making it ideal for applications requiring high uniformity, conformality, and precise thickness control (thickness is controlled at the atomic level).

TiO2 nanopillars can be also formed by:
- Electron Beam Lithography: Electron Beam Lithography (EBL) is a high-resolution patterning technique used to create extremely fine structures down to the sub-10 nm scale;
- Molecular Beam Epitaxy: Molecular Beam Epitaxy (MBE) is a highly precise thin-film deposition technique used for growing high-purity crystalline layers of semiconductors, oxides, and other materials at the atomic scale. It is widely used in semiconductor research, quantum devices, and advanced electronic/optoelectronic applications;
- ICP-Reactive Ion Etching: Inductively Coupled Plasma-Reactive Ion Etching (ICP-RIE) is an advanced dry etching technique used in semiconductor fabrication, nanotechnology, and MEMS (Micro-Electro-Mechanical Systems). It provides high anisotropy, precise etching control, and high selectivity for patterning micro- and nanoscale features.

For applications relevant to human vision, and as depicted in Figure 4, each nano-pillar 150 of the intraocular lens according to the invention has preferably a height H of between 100 and 1000 nm.

Similarly, each nano-pillar 150 has preferably a circular section S with a diameter D between 50 and 500 nm.

In the same way, the interaxial distance d between two adjacent nano-pillars is preferably in a range of 200 and 1200 nm.

In the same way, the dielectric metasurface structure comprises preferably between 10⁶ and 10⁸ nano-pillars.

In the same way, in the case of both monofocal or multifocal corrections, the plurality of nano-pillars is arranged in a range of 10³ and 10⁴ concentric rings.

Turning now to the applications under consideration, a polarization-insensitive intraocular lens according to the invention could be firstly a multifocal pseudophakic lens without stray light effects like glare and halos, thanks to the extremely shallow nano-structuration of the metasurface 140. A pseudophakic lens is defined as an artificial intraocular lens (IOL) implanted in the eye to replace the natural (crystalline) lens 201 that has been removed, usually due to cataract surgery or for refractive correction.

A polarization-insensitive intraocular lens according to the invention could secondly take the form of an ultrathin phakic lens that can be placed in the anterior 203 or posterior 208 chamber of the eye, even for important myopia corrections. A phakic lens is defined as an artificial intraocular lens (IOL) implanted in the eye without removing the natural lens 201. It is primarily used for vision correction in patients with high refractive errors, such as severe myopia (nearsightedness) or hyperopia (farsightedness), who are not candidates for LASIK or PRK.

The phakic lens according to the invention has a smaller thickness than current IOLs.

The pseudophakic lens according to the invention enable a superior control of halos, starbust and glare.

According to a third application, a polarization-insensitive intraocular lens according to the invention could consist of an IOL apt to treat specific pathologies by correcting high-order aberrations, such as keratoconus, via a personalized shaping of the light wavefront. In this case, the polarization-insensitive intraocular lens according to the invention act as a metahologram to correct optical aberrations that are beyond reach for standard IOLs.

The descriptions of the various embodiments of the present invention have been presented for purposes of illustration but are not intended to be exhaustive or limited to the embodiments disclosed. Many modifications and variations will be apparent to those of ordinary skill in the art without departing from the scope and spirit of the embodiments described. The terminology used herein was chosen to best explain the principles of the embodiments, the practical application or technical improvement over technologies found in the marketplace, or to enable others of ordinary skill in the art to understand the embodiments disclosed herein.

## Claims

1. Polarization-insensitive intraocular lens (100) comprising an anterior part (110), a posterior part (120), an equatorial plane (130), and two optical loops (190), wherein the anterior part (110) and the posterior part (120) are connected through the equatorial plane (130) and the optical loops (190) are connected with the equatorial plane (130), **characterized in that** said intraocular lens comprises moreover a solid-state dielectric metasurface structure (140), arranged on the equatorial plane (130), said metasurface structure comprising a plurality of vertical and preferably cylindrical nano-pillars (150) of different sections (S).

2. Polarization-insensitive intraocular lens according to claim 1, wherein each nano-pillar has a height (H) of between 100 and 1000 nm.

3. Polarization-insensitive intraocular lens according to any of the preceding claims, wherein each nano-pillar has a circular section (S) with a diameter (D) between 50 and 500 nm.

4. Polarization-insensitive intraocular lens according to any of the preceding claims, wherein the interaxial distance (d) between two adjacent nano-pillars is in a range of 200 and 1200 nm.

5. Polarization-insensitive intraocular lens according to any of the preceding claims, wherein the dielectric metasurface structure comprises between 10⁶ and 10⁸ nano-pillars.

6. Polarization-insensitive intraocular lens according to any of the preceding claims, wherein the plurality of nano-pillars is arranged in a range of 10³ and 10⁴ concentric rings.

7. Polarization-insensitive intraocular lens according to any of the preceding claims, wherein the plurality of vertical and preferably cylindrical nano-pillars (150) is made of Titanium Dioxide (TiO2) or of Gallium Nitride (GaN).

8. Polarization-insensitive intraocular lens according to any of the preceding claims, wherein a refractive index of each nano-pillar is in a visible wavelength range of 2.1 and 2.7.

9. Method for manufacturing a polarization-insensitive intraocular lens (100) comprising an anterior part (110), a posterior part (120), an equatorial plane (130), and two optical loops (190), the anterior part (110) and the posterior part (120) being connected through the equatorial plane (130) and the optical loops (190) being connected with the equatorial plane (130), said intraocular lens comprising moreover a solid-state dielectric metasurface structure (140) arranged on the equatorial plane (130), said metasurface structure comprising a plurality of vertical and preferably cylindrical nano-pillars (150) of different sections (S), wherein the method comprises the following steps :
- Providing a first disc (160) of solid-state biocompatible material;
- Forming on one side of said first disc the solid-state dielectric metasurface structure (140) by depositing the plurality of vertical and preferably cylindrical nano-pillars (150) of different sections (S);
- Encapsulating the solid-state dielectric metasurface structure (140) in a second disc (170) of solid-state biocompatible material, the first disc (160) and the second disc (170) being thus assembled together;
- Machining the first disc (160) and the second disc (170) according to a predefined shape to form the intraocular lens (100).

10. Method for manufacturing a polarization-insensitive intraocular lens (100) according to claim 9, wherein the step of encapsulation comprises:
- Coating the solid-state dielectric metasurface structure (140) with a liquid-state biomaterial (180);
- Covering the solid-state dielectric metasurface structure (140) coated with the liquid-state biomaterial (180), with the second disc (170) of solid-state biocompatible material.
- Curing the liquid-state biomaterial (180).

11. Method for manufacturing a polarization-insensitive intraocular lens (100) according to claim 9 or 10, wherein the plurality of vertical and preferably cylindrical nano-pillars (150) is made of Titanium Dioxide (TiO2) or of Gallium Nitride (GaN).

12. Method for manufacturing a polarization-insensitive intraocular lens (100) according to claim 11, wherein the plurality of vertical and preferably cylindrical nano-pillars (150) is formed by Electron Beam Lithography, Atomic Layer Deposition, Molecular Beam Epitaxy, or ICP-Reactive Ion Etching.

13. Method for manufacturing a polarization-insensitive intraocular lens (100) according to any of claim 9 to 12, wherein each nano-pillar has a height (H) of between 100 and 1000 nm, a circular section (S) with a diameter (D) between 50 and 500 nm, the interaxial distance (d) between two adjacent nano-pillars being in a range of 200 and 1200 nm.

14. Method for manufacturing a polarization-insensitive intraocular lens (100) according to any of claim 9 to 13, wherein the solid-state dielectric metasurface structure comprises between 10⁶ and 10⁸ nano-pillars.

15. Method for manufacturing a polarization-insensitive intraocular lens (100) according to any of claim 9 to 14, wherein the plurality of nano-pillars is arranged in a range of 10³ and 10⁴ concentric rings.

16. Method for manufacturing a polarization-insensitive intraocular lens (100) according to any one of claim 9 to 15, wherein a refractive index of each nano-pillar is in a visible wavelength range of 2.1 and 2.7.
